(19) Europäisches Patentamt / European Patent Office / Office européen des brevets

(11) **EP 2 926 131 B1**

(12) **EUROPEAN PATENT SPECIFICATION**

(45) Date of publication and mention
of the grant of the patent:
**18.07.2018 Bulletin 2018/29**

(51) Int Cl.:
***G01N 33/22*** *(2006.01)*      ***G01N 33/28*** *(2006.01)*

(21) Application number: **13792786.9**

(22) Date of filing: **29.10.2013**

(86) International application number:
**PCT/US2013/067197**

(87) International publication number:
**WO 2014/085009 (05.06.2014 Gazette 2014/23)**

(54) **METHOD FOR MODELLING THE THERMAL BREAKPOINT OF A KEROSENE FRACTION**

VERFAHREN ZUR MODELLIERUNG DES THERMISCHEN BREAKPOINTS EINER
KEROSINFRAKTION

MÉTHODE POUR MODELER LE POINT DE RUPTURE THERMIQUE D'UNE FRACTION DE
KÉROSÈNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.11.2012 US 201261731092 P**

(43) Date of publication of application:
**07.10.2015 Bulletin 2015/41**

(73) Proprietor: **ExxonMobil Research and
Engineering Company
Annandale, NJ 08801-0900 (US)**

(72) Inventors:
• **QUANN, Richard, J.**
**Moorestown, NJ 08057 (US)**
• **NOVAK, William, J.**
**Bedminster, NJ 07921 (US)**
• **QIAN, Kuangnan**
**Skilman, NJ 08558 (US)**
• **RIEDINGER, William, E.**
**Annandale, NJ 08801 (US)**
• **GAUGHAN, Roger, G.**
**Sewell, NJ 08080 (US)**
• **COODING, Beatrice, M.**
**Hopewell, NJ 08525 (US)**

(74) Representative: **ExxonMobil Chemical Europe Inc.
IP Law Europe
Hermeslaan 2
1831 Machelen (BE)**

(56) References cited:
**US-A1- 2012 153 139      US-B2- 7 297 963**

• **NICHOLAS J. KUPROWICZ ET AL: "Use of
Measured Species Class Concentrations with
Chemical Kinetic Modeling for the Prediction of
Autoxidation and Deposition of Jet Fuels",
ENERGY & FUELS, vol. 21, no. 2, 1 March 2007
(2007-03-01), pages 530-544, XP055094369, ISSN:
0887-0624, DOI: 10.1021/ef060391o**
• **XIAOBO CHEN ET AL: "Characterization and
Comparison of Nitrogen Compounds in
Hydrotreated and Untreated Shale Oil by
Electrospray Ionization (ESI) Fourier Transform
Ion Cyclotron Resonance Mass Spectrometry
(FT-ICR MS)", ENERGY & FUELS, vol. 26, no. 3,
15 March 2012 (2012-03-15) , pages 1707-1714,
XP055094466, ISSN: 0887-0624, DOI:
10.1021/ef201500r**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

FIELD OF THE INVENTION

This invention relates to a method for predicting the thermal breakpoint of kerosene fractions.

BACKGROUND

Currently, petroleum fractions used for jet fuel must satisfy the requirements specified in ASTM D1655. One of the more difficult tests to satisfy is the jet fuel thermal oxidation test as described in ASTM D3241. Unfortunately, currently available techniques have limited value in predicting whether a potential jet fuel fraction will be able to satisfy the test(s) specified in ASTM D3241. Once a fraction is found to meet all of the specifications from ASTM D1655, it is conventionally assumed that a jet fuel fraction will remain stable over time and therefore will remain within the specification limits and not need subsequent testing for requalification for use.

Conventional methods for hydroprocessing a petroleum fraction to form jet fuel rely on trial-and-error in order to identify appropriate hydroprocessing conditions. US 7297963 (MOSES ET AL.) describes a method for evaluating the thermal stability of a fuel. N.J. Kuprowicz et al., Energy & Fuel, vol. 21, 1 March 2007, pages 530-544 discloses the use of measured species class concentrations with chemical kinetic modeling to predict oxidation and deposition rates in complex flow environments for a fuel of interest, such as jet fuel. X. Chen et al., Energy & Fuel, vol. 26, 15 March 2012, pages 1707-1714 describes the characterization and comparison of nitrogen compounds in hydrotreated and untreated shall oil by electrospray ionization Fourier transform ion cyclotron resonance mass spectrometry. Such a trial-and-error process is used each time a new petroleum slate is selected for forming a feed for processing as a jet fuel.

SUMMARY

The present invention provides a method for constructing a model of the thermal breakpoint of a kerosene fraction according to claim 1.

BRIEF DESCRIPTION OF THE FIGURES

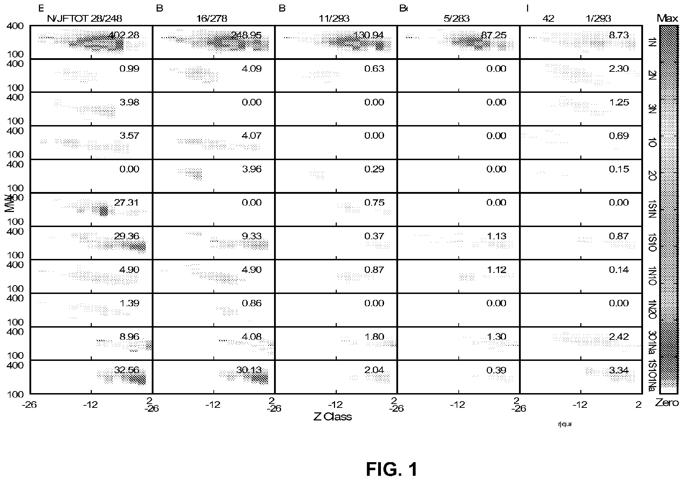

FIGURE 1 shows an example of compositional group information determined by Fourier transform ion cyclotron resonance mass spectrometry.

DETAILED DESCRIPTION OF THE EMBODIMENTS

Overview

Conventionally, the factors that cause a given kerosene sample to pass or fail a thermal breakpoint and/or thermal stability test at a given temperature are not well understood. It is known that hydrotreating of a kerosene sample tends to improve the breakpoint temperature for the sample. However, setting optimal hydrotreating conditions to allow a potential jet fuel (kerosene) feed to pass a desired thermal breakpoint and/or breakpoint stability specification has been historically a difficult trial and error process for each particular feed. For example, two kerosene feeds that appear to be similar under conventional methods of characterization can have widely different thermal breakpoint and/or breakpoint stability behavior. Due to the long time period required for at least some types of thermal breakpoint and/or stability testing, a method allowing for predication of jet fuel behavior based on common analytical techniques available in a refinery setting is desirable.

According to the present invention, a method is provided for characterizing kerosene fractions (such as potential jet fuel fractions) in order to determine whether the fractions will satisfy a desired thermal breakpoint specification. Additionally, hydrotreating conditions can be determined that will result in a hydrotreated kerosene fraction that satisfies the desired thermal breakpoint specification.

In order to predict whether a kerosene fraction will satisfy a thermal breakpoint specification and/or predict hydroprocessing conditions that will result in a hydrotreated kerosene sample that satisfies the thermal breakpoint specification, a library of data containing reference kerosene samples can be acquired using a variety of characterization methods. The data for the reference kerosene samples is preferably based on measurements performed on kerosene or jet fractions from a plurality of crude sources. Alternatively, a library can be constructed of reference kerosene samples derived from a single crude source for use in predicting properties of a specific type of kerosene fraction. A first type of data or characterization in the library can be various thermal breakpoint values for kerosene samples. For a given sample, thermal breakpoint values can be generated for a sample as initially received as well as for a sample after various amounts of hydroprocessing.

A second type of characterization corresponds to bulk physical properties for a reference sample, such as sulfur content, nitrogen content, API gravity, aromatics content, or other readily characterized values for a kerosene sample. Preferably, the characterization of physical properties can be limited to properties that are readily obtained in a non-laboratory setting, such as a refinery setting. Examples of readily obtained properties in a refinery setting include sulfur content, nitrogen content, API gravity, and/or aromatics content. It is noted that the API gravity and the aromatics content for a kerosene sample provide somewhat similar information. Therefore, it may be desirable to use only one of the API gravity and the aromatics content for a given sample. In some embodiments, the physical properties of a kerosene sample used for the second type of characterization are sulfur content, nitrogen content, and API. Other types of data that could be used in the second type of characterization include the trace metals content or the oxygen content for a kerosene sample. Currently, measurement of trace metals content or oxygen content as part of the second type of characterization is not preferred, due to the higher complexity and/or greater time required to obtain these values using conventional methods.

The above characterization values are complemented in the library by a third type of characterization of samples, which is performed using Fourier-Transform Ion Cyclotron Resonance mass spectroscopy (FTICR). Using FTICR, individual compounds within a kerosene sample can be identified both in terms of composition and quantity. This allows for a detailed qualitative and quantitative understanding of the types of molecules present in a kerosene sample, including compounds that contain heteroatoms other than carbon and hydrogen. Additionally, the changes in the amounts of these contaminant species can be investigated before and after various amounts of hydroprocessing. Based on the detailed information about the compounds within a sample, the compounds can be organized into compositional groups based on the number and type of heteroatoms in the compounds.

Another variation for using compositional information to construct a model for predicting hydrotreating conditions is to use a different definition for the compositional groups when analyzing FTICR data. For example, another way to define compositional groups is based on a "Z class" for the compositional groups. The Z class is a number based on the concept that the basic ratio of carbon to hydrogen in a hydrocarbon is one carbon per two hydrogens. The Z class represents the deviation of the ratio of carbon to hydrogen in a compound. For example, an alkane has a Z class of +2, since an alkane has a basic formula of $C_nH_{2n+2}$. A compound with one degree of unsaturation and/or one closed ring structure, such as an alkene or a single ring cycloalkane, has a Z class of zero. As more degrees of unsaturation and/or additional rings are included in a compound, the Z class will continue to decrease. For example, benzene has a Z class -6, corresponding to one ring structure plus three degrees of unsaturation. It is noted that the presence of heteroatoms may also contribute to the Z class number of a compound. A compositional group definition based on Z class can be used instead of or as a complement to the heteroatom type compositional groups described above. Particularly for a model where multiple types of crudes are used to form kerosene fractions, a large number of data points can be available. For such a model, selecting compositional groups based on both the type of heteroatoms in a compound and the Z class of compound can allow for a more refined model while still limiting the model to a manageable amount of data. Still another alternative is to not use the heteroatom identities, and instead construct compositional groups based solely on one or more Z class identifications as determined from FTICR data. Yet another alternative is to include components based on one or more individual compounds.

The detailed information from FTICR can be combined with the physical property and thermal breakpoint / breakpoint stability measurements to determine a correlation. Although FTICR provides more detailed information, the nature of FTICR makes the technique difficult to incorporate into a refinery (or other non-laboratory) setting. Instead, the FTICR information can be used to construct a model for evaluating kerosene fractions based on values that are more readily obtained, such as breakpoint temperature and other bulk compositional/physical properties.

For example, the thermal breakpoint, bulk physical properties, and FTICR data for a plurality of reference samples can be stored for use in a model. The plurality of reference samples preferably include at least some samples that are related based on the samples being derived from the same kerosene source. However, these related samples can still differ based on the amount and/or severity of hydrotreatment performed on the sample.

When the model is used for prediction of the amount of hydroprocessing necessary for a new sample, the new sample is initially compared to the plurality of samples already in the database. This comparison is made, for example, based on the initial nitrogen, sulfur, API (and/or aromatics), and thermal breakpoint values for the new sample. By comparing with existing samples, a set of FTICR information is predicted for the new sample. The predicted FTICR information can then be used along with the nitrogen, sulfur, and API (and/or aromatics) values to select hydroprocessing conditions that will result in a thermal breakpoint / breakpoint stability for the hydroprocessed product that will satisfy the requirements in ASTM D1655.

Stability Testing for Proposed Jet Fuel Products

In the discussion herein, references to a "breakpoint" or "thermal breakpoint" are references to a JFTOT™ type thermal breakpoint as defined by ASTM D3241. (JFTOT refers to a jet fuel thermal oxidation test defined in ASTM D3241. JFTOT is currently a registered trademark of Petroleum Analyzer Company.) Jet fuel products are typically qualified, with regard to thermal stability, using an ASTM standard test (ASTM D3241) to determine if the product properties satisfy the thermal stability specifications in ASTM D1655. The ASTM D3241 test is a "pass/fail" type test, meaning that a proposed jet fuel fraction is either qualified or not qualified for use. Similarly, references to a "breakpoint stability" are references to a JFTOT™ type breakpoint stability, as understood with reference to ASTM D3241 and/or ASTM D1655.

Jet fuel products are generally tested using thermal breakpoint testing procedures that are defined in ASTM D3241. The test involves flowing a jet fuel sample in an elevated temperature environment over a metal heater tube under specified conditions. For example, a jet fuel sample can be passed from a reservoir over a metal heater tube at a temperature of 265°C and at a pressure of about 500 psig (3.44 MPag). The output from the metal heater tube is then passed through a differential pressure filter. The flow rate from the reservoir is typically maintained at a constant value, such as 3.0 ml/min for a set period of time, such as 150 minutes. After the test, the deposits on the metal heater tube are evaluated for color and pattern. This establishes a "tube rating" for the test. The maximum pressure drop across the filter is also determined. A proposed jet fuel sample is deemed to pass the test if both the tube rating and pressure drop values are satisfactory.

One option is to test a jet fuel sample at a single temperature, such as 265°C, to qualify the sample for use. Common temperatures used as a target temperature for qualifying a sample for use include 260°C, 265°C, 270°C, 275°C, or 280°C. Another option is to determine a breakpoint for the sample. To identify a breakpoint, a series of tests are performed at temperatures that differ by an interval of 5°C. At lower temperatures, the jet fuel sample will pass the tube rating (deposits) and pressure drop tests. As the temperature is increased, a temperature interval will eventually be reached where the sample has satisfactory tube rating and pressure drop values at the temperature on the lower side of the interval while failing one or both of the tube rating and pressure drop portions of the test on the high temperature side of the interval. The lower temperature of the pair of temperatures corresponding to the interval is defined as the breakpoint for the sample. In other words, the breakpoint temperature is a temperature where any further temperature increase is likely to result in failure of the sample to pass the test defined in ASTM D3241.

The breakpoint for a potential jet fuel or kerosene sample can be determined at various times. One time period for determining a thermal breakpoint is prior to hydroprocessing, while another time is after some amount of hydroprocessing. Still another time for determination of a breakpoint is after a sample has aged. This allows the method for determining a breakpoint temperature to be expanded to provide an improved method for determining the breakpoint stability of a sample. Although some jet fuel is used relatively soon after production at a refinery, it is desirable for a jet fuel sample to be stable for periods of a year or even longer. The breakpoint stability of an aged sample can be tested by determining whether an aged sample still satisfies a desired breakpoint specification.

Of course, one method for determining whether a potential jet fuel remains suitable for use after aging for a year is to use a brute force method. For example, a jet fuel can be stored for six months or a year at 20°C and then tested using an appropriate ASTM method. While this type of aging allows for accurate determination of breakpoint stability, the time required for this type of testing is not usually practical. Another option is to use some type of accelerated aging, such as by aging a sample at a temperature greater than 20°C for a shorter period of time. For example, aging a sample at about 43°C for a week is considered to be equivalent to aging a sample at 20°C for a month. (See ASTM D4625.) Thus, accelerated aging can be performed by storing a sample at 43°C (or another suitable temperature of at least about 40°C) for a desired period of time. Aging for a period of at least about 6 weeks roughly corresponds to aging for 6 months, while aging for a period of 12 weeks roughly corresponds to aging for a year. After aging, the breakpoint temperature for the aged sample can be determined using an appropriate ASTM method, in order to evaluate the breakpoint stability of the sample.

In addition to verifying that an aged sample meets a breakpoint temperature specification, the amount of change in breakpoint temperature after aging can also be valuable. After aging, a sample may have a breakpoint temperature that, from an absolute value standpoint, still satisfies a desired breakpoint temperature specification. However, a sharp drop in breakpoint temperature for a sample after aging indicates a sample that may be unreliable after storage. Thus, in addition to the absolute breakpoint temperature for an aged sample, in some embodiments a kerosene fraction can be characterized as having an unsatisfactory breakpoint stability over time when the breakpoint for the sample changes by more than 10°C after 1 year of storage and/or under conditions that simulate a year of storage at standard temperature of about 20°C. Alternatively, a kerosene fraction having an unsatisfactory breakpoint stability can correspond to a kerosene fraction where the breakpoint changes by more than 6°C after 6 months of storage and/or under conditions that simulate 6 months of storage.

For example, a sample with a breakpoint of 275°C before aging and a breakpoint of 265°C after aging for 12 weeks at 43°C is still suitable for use as a jet fuel, even though the breakpoint for the sample has decreased. In this situation, the breakpoint of the sample has changed by 10°C or less during the equivalent of aging for 1 year. By contrast, a sample with a breakpoint of 280°C before aging and a breakpoint of 265°C after aging for 12 weeks at 43°C may or may not be suitable for use as a jet fuel. In this example, the breakpoint of the aged sample still satisfies the ASTM D3241 breakpoint requirement. However, the degradation of the breakpoint by 15°C during the equivalent of aging for 1 year may indicate a sample that will continue to degrade in an unacceptable manner.

More generally, sample stability can be tested by first determining a breakpoint for jet fuel product samples by increasing the testing temperature for samples of the potential product. After identifying the break point, one or more samples of the jet fuel product can be aged at a temperature above 40°C for at least 6 weeks, such as for at least 10 weeks or at least 12 weeks. Examples of suitable testing temperatures are 43°C as described in ASTM D4625, 65°C as described in CRC report CA-43-98, or 95°C as described in ASTM D2274. Preferably, the aging temperature is about 43°C. After aging, the breakpoint for an aged sample of the jet fuel product is determined again to verify that the jet fuel product sample still passes the tube rating and pressure drop tests at a sufficiently high temperature to qualify for use as a jet fuel product.

Feedstocks and Hydroprocessing

In some embodiments, a feedstock for forming a jet fuel corresponds to a fraction of a crude oil that has a suitable boiling range. One example of a suitable boiling range for a jet fuel is a fraction with an initial boiling point of least about 284°F (140°C) and a final boiling point of less than about 572°F (300°C). A feedstock for forming a jet fuel may have a narrower and/or higher boiling range, to account for any conversion of a feedstock that may occur during hydroprocessing. The sulfur content of a suitable jet fuel fraction is 3000 wppm or less, such as about 1500 wppm or less or about 500 wppm or less.

Traditionally, kerosene fractions used for jet fuel have been derived from mineral crudes. Alternative crude sources such as pre-refined crudes have been avoided due to uncertainties about the breakpoint stability of such feeds. In some embodiments, kerosene fractions derived from such alternative sources may be included as part of a jet fuel. Potential alternative sources include, but are not limited to, kerosenes derived from pre-refined crudes and/or derived from biological sources.

Traditionally, fractions derived from a cracking type process, such as an output stream from a fluid catalytic cracking unit, a coker, or a visbreaking unit, have not been used for forming a jet fuel. Conventionally, it is believed that petroleum fractions derived from a cracking process will have poor stability and therefore will not be able to satisfy JFTOT requirements after long periods of storage. In some embodiments, kerosene fractions derived from cracking type processes may be included as part of a jet fuel. Potential sources include, but are not limited to, kerosenes derived from outputs of cracking processes, such as fluid catalytic cracking processes, coking processes, or other thermal cracking processes such as visbreaking.

One option for upgrading a kerosene fraction (such as a kerosene fraction corresponding to a potential jet fuel fraction) is to hydroprocess the kerosene fraction, such as by hydrotreatment. A wide range of hydroprocessing conditions are potentially suitable for use, as even mild hydroprocessing conditions may produce a benefit in the properties of the jet fuel fraction. During hydroprocessing, a feedstock that is partially or entirely composed of a jet fuel boiling range fraction is treated in a hydrotreatment (or other hydroprocessing) reactor that includes one or more hydrotreatment (or other hydroprocessing) stages or beds. Optionally, the reaction conditions in the hydrotreatment stage(s) can be conditions suitable for reducing the sulfur content of the feedstream, such as conditions suitable for reducing the sulfur content of the feedstream to about 3000 wppm or less, or about 1000 wppm or less, or about 500 wppm or less. Additionally or alternately, the reaction conditions in the hydrotreatment stages can be conditions suitable for improving the JFTOT breakpoint temperature for a sample independent of or as a complement to any reduction in the sulfur content. The reaction conditions can include an LHSV of 0.1 to 20.0 hr$^{-1}$, a hydrogen partial pressure from about 50 psig (0.34 MPag) to about 3000 psig (20.7 MPag), a treat gas containing at least about 50% hydrogen, and a temperature of from about 400°F (204°C) to about 800°F (427°C). Preferably, the reaction conditions include an LHSV of from about 0.3 to about 5 hr$^{-1}$, a hydrogen partial pressure from about 100 psig (0.69 MPag) to about 1000 psig (6.9 MPag), and a temperature of from about 450°F (232°C) to about 600°F (316°C). Preferably, the hydrotreatment conditions selected for treating a potential jet fuel fraction are selected to be sufficiently severe to satisfy desired specifications without over-processing the fraction. Hydroprocessing a potential jet fuel fraction beyond the minimum amount necessary to meet a specification will typically still result a product the meets desired specifications, but in a lower yield.

Optionally, a hydrotreatment reactor can be used that operates at a relatively low total pressure values, such as total pressures less than about 800 psig (5.5 MPag). For example, the pressure in a stage in the hydrotreatment reactor can be at least about 200 psig (1.4 MPag), or at least about 300 psig (2.1 MPag), or at least about 400 psig (2.8 MPag), or at least about 450 psig (3.1 MPag). The pressure in a stage in the hydrotreatment reactor can be about 700 psig (4.8 MPag) or less, or about 650 psig (4.5 MPag) or less, or about 600 psig (4.1 MPag) or less.

The catalyst in a hydrotreatment stage can be a conventional hydrotreating catalyst, such as a catalyst composed of a Group VIB metal (Group 6 of IUPAC periodic table) and/or a Group VIII metal (Groups 8 - 10 of IUPAC periodic table) on a support. Suitable metals include cobalt, nickel, molybdenum, tungsten, or combinations thereof. Preferred combinations of metals include nickel and molybdenum or nickel, cobalt, and molybdenum. Suitable supports include silica, silica-alumina, alumina, and titania.

In an embodiment, the amount of treat gas delivered to the hydrotreatment stage can be based on the consumption of hydrogen in the stage. The treat gas rate for a hydrotreatment stage can be from about two to about five times the amount of hydrogen consumed per barrel of fresh feed in the stage. A typical hydrotreatment stage can consume from about 50 SCF/B ($8.4 \ m^3/m^3$) to about 1000 SCF/B ($168.5 \ m^3/m^3$) of hydrogen, depending on various factors including the nature of the feed being hydrotreated. Thus, the treat gas rate can be from about 100 SCF/B ($16.9 \ m^3/m^3$) to about 5000 SCF/B ($842 \ m^3/m^3$). Preferably, the treat gas rate can be from about four to about five time the amount of hydrogen consumed. Note that the above treat gas rates refer to the rate of hydrogen flow. If hydrogen is delivered as part of a gas stream having less than 100% hydrogen, the treat gas rate for the overall gas stream can be proportionally higher.

Characterization of Kerosene Samples with FTICR

Kerosene or jet fuel boiling range samples can be characterized using a variety of techniques. As noted above, breakpoint and/or breakpoint stability can be measured for a kerosene or jet fuel fraction using appropriate ASTM methods. Compositional analysis can also be performed, such as analysis to determine the sulfur content, nitrogen content, and API gravity (and/or aromatics content) of a sample.

FTICR is used to complement the information derived from the above characterization techniques. Briefly, FTICR is a particular type of mass spectrometry that allows for detailed resolution of the composition of a sample. Unlike many types of mass spectrometry, an ion cyclotron resonance mass spectrometer does not detect species based on collisions with a detector. Instead, after forming ions from the species in a sample, the ions are trapped within the magnetic field, resulting in a cyclotron as the ions traverse an (approximately) circular path within the magnetic field. The speed of each ion varies depending on the mass at a given energy. This speed differences allows the electric field generated by different ions traveling in the magnetic field to be detected and distinguished. This time-domain electric signal is converted by Fourier transform into frequency-domain signals that correspond to the different types of ions in the magnetic field. This allows for detailed differentiation between the compounds within a sample.

FIG. 1 shows an example of the types of compositional details that can be identified using FTICR mass spectrometry. FIG. 1 shows output data from performing FTICR on a kerosene boiling range sample. In FIG. 1, several different types of axes are shown. Each row in FIG. 1 corresponds to a compositional class of compounds categorized based on the number and type of heteroatoms, such as 1 nitrogen (top row) or 2 nitrogens, an oxygen, and a sodium (bottom row). Each column in FIG. 1 corresponds to the kerosene sample after various amounts of hydrotreating. The first column corresponds to a raw kerosene cut before hydroprocessing. The unprocessed kerosene fraction has a nitrogen content of 28. As the columns move to the right, the columns show the same type of kerosene cut after successively larger amounts of hydrotreatment. As a result, the amount of total nitrogen in the kerosene cuts decreases moving to the right in the table.

Each box also has an individual horizontal and vertical axis. The horizontal axis for each individual box corresponds to the Z class of the molecules in the box. The vertical axis within each box represents the molecular weight of the compounds within the box. Optionally, a signal intensity within the box can be used to represent the number of compounds for a given combination of Z class and molecular weight.

As shown in FIG. 1, the compositional data available from FTICR provides an opportunity to characterize compounds within a kerosene fraction at a wide range of granularities, including characterization of compounds as individual compounds or as a member of various types of compositional classes. In particular, the amounts of each type of impurity molecule, such as species containing nitrogen, sulfur, oxygen, and or sodium, can be characterized. The characterization can further include grouping of compounds based on molecular weight, such as by molecular weight within a compositional class.

The detailed compositional information generated using FTICR is used to build a model that correlates the detailed compositional information with measured thermal breakpoint and breakpoint stability values. As an initial step in building a model, the sulfur, nitrogen, API (or aromatics), and optionally other physical properties of kerosene sample are obtained, along with thermal breakpoint and/or breakpoint stability values for the sample. The sample is also characterized using FTICR, to obtain detailed compositional information as shown in FIG. 1. It is noted that the FTICR data will preferably include information beyond the common types of physical properties that can be readily obtained in a refinery setting. For example, the physical properties may include only the sulfur, nitrogen, and API (or aromatics) contents for a sample. These physical property values represent bulk values for the sample, and do not provide further insight about how functional groups are arranged within compounds in the sample. By contrast, the FTICR data can expand both the types of atoms that are characterized and the amount of detail available for the characterized atoms. For example, the results from an FTICR analysis can also include information about the presence of sodium and oxygen. Additionally, the FTICR data can provide more detailed information about how nitrogen, sulfur, aromatics, sodium and/or oxygen are distributed in the various compounds within a sample. This initial step can be repeated for a plurality of samples. The samples can include raw or virgin kerosene samples as well as kerosene samples after some amount of hydrotreatment. Preferably at least some of the samples are related in the sense of being derived from the same kerosene source but differing based on the amount of hydrotreatment the sample has been exposed to. By obtaining a plurality of samples, a library can be accumulated of reference samples.

Optionally but preferably, at least a portion of the reference samples can also include information about whether an aged version of the sample satisfies the specifications in ASTM D1655 for breakpoint stability. If sufficient information about aged samples is present, the model can be used to predict hydroprocessing conditions that allow hydroprocessed sample to satisfy both the thermal breakpoint and breakpoint stability requirements.

The total nitrogen and thermal breakpoint values shown in FIG. 1 provide an example of the difficulties associated with building a model for predicting breakpoint values without having the detailed compositional information provided by FTICR. As shown in FIG. 1, increasing the amount of hydrotreatment decreases the amount of nitrogen remaining in a kerosene sample in a somewhat regular manner. However, the thermal breakpoint temperature of the sample does not increase monotonically with decreasing nitrogen content and/or with increasing hydroprocessing time. For example, for a kerosene sample (1st column) with an initial nitrogen content of 28 wppm and an initial thermal breakpoint of 248°C, an intermediate amount of hydrotreatment (3rd column) results in a breakpoint temperature of 293°C and a nitrogen content of 11 wppm. Further hydrotreatment (4th column) reduces the nitrogen content to 5 wppm, but the thermal breakpoint drops to 283°C. Still more hydrotreatment reduces the nitrogen content to 1 wppm and returns the thermal breakpoint to 293°C. This demonstrates that the correlation between nitrogen and/or hydroprocessing severity is not a simple linear relationship. By using FTICR, information at a more detailed level of granularity is available. This allows for construction of a model based on compositional groups within a sample, and therefore also allows components to be included in a model that represent how the interaction of various compositional groups influences the thermal breakpoint for a sample.

Constructing a Model using Compositional Information

In various embodiments, one goal is to use a hydroprocessing model to predict the hydroprocessing conditions that are needed to achieve a desired thermal breakpoint for a kerosene sample. Such a hydroprocessing model can include a plurality of components. Based on empirical data from prior FTICR analysis of kerosene samples (or other techniques for determining detailed composition information), the model can include a plurality of components that correspond to various compounds that may be present within a kerosene sample. As shown in the FTICR example in FIG. 1, compounds containing a nitrogen atom and a sulfur atom or other combinations of heteroatoms can be detected. This composition information can be used to assign values to the corresponding components in a model. The components can represent compounds that contain various heteroatoms or other functional groups that are identified based on FTICR data, such as compounds that include one or more nitrogen atoms, sulfur atoms, sodium atoms, oxygen atoms, aromatic rings, and/or non-aromatic rings. These compounds can be represented by the components as individual compounds, as compositional groups, as groups based on Z class, or in any other convenient method.

The model can be thought of as corresponding to two types of relationships between data. A first relationship describes a relationship between thermal breakpoint, physical properties that are readily measured, and FTICR data values. This relationship can be described conceptually as

$$(1)\ N + API\ (\text{or aromatics}) + S + FTICR\ \longleftrightarrow\ \text{thermal breakpoint}$$

The above conceptual relationship can be captured in the model by using a plurality of components to represent the measured values in various ways. Some of the components can represent the influence of the physical properties on the thermal breakpoint of a sample. Other components can represent the influence of individual compounds and/or compositional groups on thermal breakpoint. Still other components can represent how the interaction of compounds and/or compositional groups with each other impacts the thermal breakpoint. Coefficients for the various components can be fit so that the model matches measured values for reference samples. Relationship (1) can also be used to predict the components corresponding to FTICR data if the nitrogen, API, sulfur, and thermal breakpoint values for a sample are known.

Based on the empirical data, the model can also include a second type of relationship describing how components are modified based on various hydroprocessing conditions. A relationship can be determined for how each type of individual compound and/or compositional group represented in the model is changed during hydroprocessing, and additional components can be added to the model to describe this change. The components related to how hydroprocessing impacts the compositional groups in the model can be derived from the reference library, or this information can be derived from characterization of other samples independent of the reference library. This second type of relationship can be described conceptually as

## (2) N + API + S + FTICR$_{(est.)}$ + Hydroprocessing Model\* <--> thermal breakpoint$_{(product)}$

This second relationship can be used to predict the hydroprocessing conditions needed to achieve a desired thermal breakpoint. Depending on the embodiment, a hydroprocessing model can include thousands of components to represent the various interactions that occur within a kerosene sample to determine thermal breakpoint, and to further capture the impact of hydroprocessing on the compounds / compositional groups with a kerosene sample. As an example, a suitable model for correlating thermal breakpoint with compositional group data and capturing the impact of hydroprocessing on the compositional groups can potentially include thousands of components. Such a model could include about 500 components or more that represent the detailed compositional information that is provided by FTICR for compounds and/or compositional groups.

As noted above, the parameters or coefficients for the components in the model can be fit to combinations of measured initial thermal breakpoint values, compositional information prior to hydroprocessing, hydroprocessing conditions, thermal breakpoint values after hydroprocessing, and compositional information after hydroprocessing. The combinations of measured values are based on the library of measured or characterized reference kerosene samples.

As an example, several types of characterization can be performed on the kerosene fractions corresponding to reference samples prior to (and optionally after) hydrotreatment. Some or all of these characterizations can be performed on each available sample. The characterizations include determination of thermal breakpoint according to ASTM 3241 and/or ASTM D1655; determination of thermal breakpoint for a sample aged according to ASTM D4625 or D2274; determination of total sulfur, nitrogen, and API gravity and/or aromatics; and determination of compositional groups based on FTICR. For the determination of compositional groups, in this example the compounds in a sample are classified based on the following groups: 1 N (meaning one nitrogen); 2 N; 3 N; 1 O (oxygen); 2 O; 1 N plus 1 S (sulfur); 1 S plus 1 O; 1 N plus 1 O; 1 N plus 2 O; 2 O plus 1 Na (sodium); 3 O plus 1 Na; 4 O plus 1 Na; 1 S plus 1 O plus 1 Na; and 1 N plus 2 O plus 1 Na. Other molecules are classified in an "other category" but are not directly of interest for the construction of the model. The components based on compositional groups in the model can then be determined based on the amounts of each compositional group in the model, cross-terms representing the interaction of two or more compositional groups, or any other component that is convenient to define. In alternative embodiments, one or more other compositional groups could be used in place of or in addition to the specified listing above, and/or one or more of the above compositional groups could be eliminated to reduce the overall number of compositional groups. In this example, compositional groups based on a particular Z class are not used, but groups based on Z class can be used in place of or in addition to other types of compositional groups. Still another option is to include components that are based on individual compounds, as opposed to just using components based on compositional groups and/or Z classes.

After constructing a hydroprocessing model for a kerosene fraction, the model can be used to predict hydroprocessing conditions that are needed to achieve a desired final thermal breakpoint value. As described above, the library of kerosene samples includes a plurality of samples with measured sulfur, nitrogen, API, thermal breakpoint values, and FTICR compositional data. Optionally, the FTICR compositional data can already be converted to be in the form of the components for the model. Alternatively, the FTICR compositional data can be in a raw form, and then used to construct component values after assignment of FTICR values to a new reference compound. Optionally but preferably, the plurality of samples can also include breakpoint stability values. This library can be used as a series of reference points.

When a prediction of the amount of hydroprocessing for a kerosene sample is desired, physical properties (such as sulfur, nitrogen, and API gravity) can be obtained for the new sample along with a thermal breakpoint and/or breakpoint stability. These measured properties can then be compared with the reference samples in the library. If the measured properties for the new sample match the properties for a reference sample, the FTICR compositional data / components for the reference sample can be assigned to the new sample. If there is not a sufficiently close match, FTICR values can be assigned to the new sample based on some type of average of the properties of reference samples that have similar properties to the new sample. For example, a group of two or more nearest neighbor reference samples can be identified. Reference samples can be identified as nearest neighbors based on a similarity of the thermal breakpoint, sulfur content, nitrogen content, aromatics content, and/or API gravity of a reference sample as compared to the corresponding measured values for the new sample. Any convenient threshold value can be used for determining that a value for a reference sample is similar to a value for the new sample. For example, a threshold can be based on a percentage of the value for the new sample, so that a reference sample with a value that differs by less than 5%, such as by less than 2%, is considered a nearest neighbor. Optionally, a reference sample can be required to have at least two values that fall within the threshold in order to qualify as a nearest neighbor for a new sample. Optionally, a limited number of reference samples can be selected as nearest neighbors based on a similarity for any given value. For example, the number of reference samples identified as nearest neighbors based on thermal breakpoint can be limited to the closest two thermal breakpoint values less than the measured value for the new sample and the closest two thermal breakpoint values greater than the measured value for the new sample. More generally, any convenient method can be used to identify reference samples that are sufficiently similar to the new sample based on the measured values for the new sample.

The nearest neighbor reference sample values can then be combined in some type of weighted average or by interpolation to provide a group of properties that roughly matches the measured properties for the new sample. Preferably, the combination of the reference samples results in a weighted average of the thermal breakpoint and the physical properties for the average of reference samples that differs from the corresponding measured properties of the new sample by less than a tolerance value, such as differing by less than 5% for each measured property for the new sample. Based on the coefficients of the weighted average and/or the interpolation, a corresponding set of compositional data / components can be assigned to the new sample. Without the reference library of samples that have FTICR data to provide compositional information / components, this assignment of component values to a new sample would not be possible.

One of the benefits of being able to assign the compositional information / component values based on the reference library is that more detailed compositional information can be associated with a new sample. For example, the FTICR compositional data can include information about the weight percentage of species containing oxygen or sodium heteroatoms. Without the reference library of samples, a simple measurement of sulfur, nitrogen, API gravity and/or aromatics, and thermal breakpoint would not provide any information about such additional heteroatoms. Additionally, the FTICR information associated with the new sample provides more detailed information about the specific nature of what types of species contain heteroatoms, such as the correlation between heteroatoms and aromatics and/or Z class.

After assigning the compositional values / components corresponding to FTICR type data to a new reference compound, the thermal breakpoint value does not need to be used during the prediction of the hydroprocessing conditions. Instead, the nitrogen content, sulfur content, API gravity (or aromatics content), and the assigned component values (optionally calculated from compositional values) are used as inputs for determining the hydroprocessing conditions that will cause the sample to satisfy a desired thermal breakpoint. Preferably, the hydrotreating conditions determined from the model will also correspond to conditions that produce a kerosene sample with a desired breakpoint stability, based on the ability of an aged sample to satisfy a thermal breakpoint specification and/or the ability of the aged sample to have a thermal breakpoint temperature that is sufficiently similar to the thermal breakpoint of the sample prior to aging.

The scope of the model can be tailored to match the available reference samples. One option is to build a model for kerosene fractions derived from a single crude source. For example, whole or partial crudes extracted from a single mineral source, and that undergo similar pre-processing at an extraction site, are typically expected to have similar compositional profiles. Thus, kerosene fractions derived from a single crude source would also be expected to have a similar profile. As a result, it is expected that a model can be constructed by acquiring a library of data based on the single crude source. The library can include thermal breakpoint information, breakpoint stability information, and FTICR compositional data for raw kerosene fractions derived from a crude source and corresponding hydrotreated kerosene fractions after various amounts of hydrotreatment. Preferably, other data such as total sulfur, total nitrogen, API gravity and/or aromatics content, and/or other characterization data can also be included.

Selection of Hydroprocessing Conditions for Kerosene Fractions

Once a model is constructed, several options are available for using the model. For example, after building the model, a new kerosene fraction can be distilled or otherwise separated from a new delivery of the whole or partial crude from the identified source. In a laboratory setting, one option would be to use FTICR to characterize the new kerosene fraction. A thermal breakpoint could also be determined. Based on the FTICR data, the components corresponding to compositional groups could be directly determined. The amount of hydroprocessing needed to meet a desired breakpoint specification can be predicted based on the model. The new kerosene fraction could then be hydrotreated according to the conditions calculated from the model. By selecting hydrotreatment conditions based on the model, the hydrotreating conditions can be selected to generate a hydrotreated kerosene product that satisfies a desired thermal breakpoint specification while using a minimum hydrotreating severity. Of course, the thermal breakpoint and/or breakpoint stability of the hydrotreated kerosene fraction can be verified by testing according to an appropriate ASTM method.

While the above laboratory setting method is effective, most refineries do not currently have access to the equipment necessary for performing FTICR characterization of a sample. Thus, it would be preferable to be able to employ the model based on values that can be obtained more easily, such as based on only an initial thermal breakpoint, a nitrogen content, a sulfur content, and an API gravity (and/or aromatics content).

In situations where measured FTICR data is not available for a new sample, the hydroprocessing conditions can be predicted by first using the model to select or derive component values for the FTICR type compositional data in the model that can be assigned to the new sample. As described above, the measured thermal breakpoint, nitrogen content, sulfur content, and API gravity (and/or aromatics content) can be used to select (and optionally calculate) component values for components in the model based on compositional groups and/or individual compounds. In effect, the thermal breakpoint, nitrogen content, sulfur content, and API gravity are used to select / predict a likely set of FTICR data. The FTICR data does not need to correspond to all data that would be generated during an FTICR test. Instead, the data can just correspond to the compositional group data needed for determining the components in the model. This selection or prediction is made by identifying one or more similar reference samples in the reference library, and then using some type of interpolation or linear combination to match the nearest neighbor samples with the new sample. The same interpolation or linear combination is then applied to the component values for the nearest neighbor reference sample(s) to derive component values for assignment to the new sample.

After selecting (including deriving) components for the FTICR type data in the model to assign to the new sample, the nitrogen content, sulfur content, API gravity, and assigned components are used to predict the hydroprocessing conditions to achieve the desired thermal breakpoint. The initial thermal breakpoint is implicitly included in the selected components, so the thermal breakpoint does not need to be included when predicting the hydroprocessing conditions. The model is then used to predict the type of hydroprocessing conditions that are needed to reduce the component values to a level so that the component values, sulfur, nitrogen, and API gravity correspond to a reference sample (or combination of reference samples) that will satisfy the desired thermal breakpoint. The predicted hydroprocessing conditions can include a hydroprocessing temperature, a hydroprocessing pressure, a length of hydroprocessing time (such as space velocity), and/or any other convenient conditions. If desired, the model can be used in a manner where one or more hydroprocessing conditions remain fixed at a default value while the other values are selected to achieve a desired thermal breakpoint. For example, a default pressure and/or a default catalyst activity can be selected, so that determining the hydroprocessing conditions corresponds to selecting a reaction temperature and a run length.

It is noted that this second type of situation is still referred to above as using a single, unified model. However, whether the model is considered a single model, a pair of models, or a plurality of models is not critical. Regardless of how the model(s) are viewed, the FTICR individual compound and/or compositional group data is the factor that allows for prediction of thermal breakpoint values for a kerosene sample. Thus, even though different functional forms may be used in a laboratory versus a refinery setting, the description herein will refer to these as a single model for predicting hydrotreatment conditions to produce kerosene with desired thermal breakpoint specifications.

With regard to modeling kerosene fractions to predict hydrotreatment conditions, it is preferable to initially construct the model using a database that is representative of how the model will be used. Constructing a model for use with kerosene fractions from a single crude source is an example of this. A model constructed based on data from a single crude source should be effective for predictions for other kerosene fractions from the same crude source. However, limiting the model to a single crude source limits the value of the model, as a new model needs to be developed each time a new crude source is considered.

Expanding the model to incorporate kerosene fractions derived from multiple crude sources can greatly expand the value of the model. However, it is still desirable to have a model that is commensurate in scope with the types of kerosene fractions that the model will be applied to. For example, if kerosene fractions from only conventional (mineral) fractions are used to construct the model, the performance of the model may be questionable when applied to kerosene fractions derived from non-traditional sources, such as biological sources or pre-refined crudes. This does not mean that a model constructed based on conventional kerosene fractions cannot be applied to other types of fractions. Rather, if the model is not commensurate in scope with the kerosene fractions it is applied to, there may be greater uncertainty about the predicted hydrotreating conditions.

A model that incorporates kerosene fractions based on multiple crude sources can be developed in a manner similar to a model for a single crude source. For initial construction of the model, kerosene fractions derived from a plurality of crude sources are characterized before hydrotreatment and after various amounts of hydrotreatment. The kerosene fractions are characterized for thermal breakpoint and optionally breakpoint stability. The kerosene fractions are also characterized using FTICR to identify compositional groups in the kerosene fractions, as described above. Additionally, the kerosene fractions are characterized for other physical properties, such as sulfur content, nitrogen content, aromatics content, and/or API gravity.

In a first portion of the model, the compositional groups are used in combination with the breakpoint and other characterization data to generate a model for predicting breakpoint values. A second portion of the model can also be created based on a more limited set of observables, such as model based on sulfur, nitrogen, aromatics, and initial thermal breakpoint temperature. As described above, this second model may include non-linear terms and/or terms involving multiple compositional variables. The model can then be used to predict the hydrotreating conditions needed to produce a hydrotreated kerosene product that satisfies a desired thermal breakpoint specification.

Variations on Compositional Model - Model Feedback and Alternative Compositional Groups

As noted above, after initial construction of a model, it is expected that predictions of hydrotreating conditions will be based on data other than FTICR measurements. When hydrotreatment is performed, the hydrotreated kerosene can be characterized for thermal breakpoint and basic compositional information, such as total sulfur, nitrogen, aromatics, and/or API gravity. As a result, additional thermal breakpoint and basic compositional data will be generated without corresponding FTICR measurements. This additional data can be used to provide feedback for the model.

During use of the model, an initial thermal breakpoint will be determined for a kerosene fraction. Additionally, at least a sulfur content, nitrogen content, and API gravity (and/or aromatics content) will also be determined for the kerosene fraction. After hydrotreatment, a thermal breakpoint value and the sulfur, nitrogen, API gravity, and/or aromatics can be determined for the hydrotreated kerosene. This additional information is not used to modify the format of the model, but it can be used to update coefficients. For example, one or more additional sets of data can be incorporated by assigning the additional data sets an appropriate weight relative to the existing coefficients for the model. As with determination of an initial fit for coefficients for the model, a variety of options are available for updating the model coefficients.

**Claims**

1. A method for constructing a model of the thermal breakpoint of a kerosene fraction, comprising:

   measuring a thermal breakpoint for a plurality of kerosene fractions;
   measuring one or more of a sulfur content, a nitrogen content, an aromatics content, and an API gravity for the plurality of kerosene fractions;
   analyzing the plurality of kerosene fractions, using Fourier transform ion cyclotron resonance mass spectrometry, to determine weights for a plurality of compositional groups within each kerosene fraction; and
   constructing a model for correlating a thermal breakpoint of a kerosene fraction with a) the one or more of a sulfur content, a nitrogen content, an aromatics content, and an API gravity for a kerosene fraction, and b) weights for a plurality of compositional groups in a kerosene fraction, the model being constructed based on the measured thermal breakpoints for the plurality of kerosene fractions, the measured values for the one or more of a sulfur content, a nitrogen content, an aromatics content, and an API gravity for the plurality of kerosene fractions, and the determined weights for the plurality of compositional groups for the plurality of kerosene fractions.

2. A method for preparing a kerosene product, comprising constructing a model of the thermal breakpoint of a kerosene fraction as recited in claim 1, and further comprising:

   determining, for a first kerosene fraction, a thermal breakpoint and the one or more of a sulfur content, a nitrogen content, an aromatics content, and an API gravity;
   using the model to assign weights for a plurality of compositional groups for the first kerosene fraction based on the determined thermal breakpoint and the determined one or more of a sulfur content, a nitrogen content, an aromatics content, and an API gravity;
   selecting hydroprocessing conditions for the first kerosene fraction based on the assigned weights for the plurality of compositional groups, the determined one or more of a sulfur content, a nitrogen content, an aromatics content, and an API gravity, and the constructed model; wherein the model includes data relating to samples of kerosene fractions which have been hydroprocessed and also includes a relationship describing how compositional groups are modified based on various hydroprocessing conditions; and
   hydroprocessing the first kerosene fraction using the selected hydroprocessing conditions.

3. The method of claims 1 or 2, wherein the plurality of kerosene fractions are derived from a single oil source.

4. The method of any of the above claims, wherein one or more of the plurality of kerosene fractions are derived from a biological source, a pre-refined crude oil source, or an oil source that has been exposed to a cracking process, or wherein one or more kerosene fractions in the plurality of kerosene fractions comprise aged kerosene fractions, or a combination thereof.

5. The method of any of the above claims, wherein one or more kerosene fractions in the plurality of kerosene fractions are hydrotreated kerosene fractions, a plurality of the one or more hydrotreated kerosene fractions preferably being related to at least one other kerosene fraction in the plurality of kerosene fraction based on being derived from the same oil source.

6. The method of any of the above claims, wherein measuring one or more of a sulfur content, a nitrogen content, an aromatics content, and an API gravity for the plurality of kerosene fractions comprises measuring a sulfur content, a nitrogen content, and an API gravity.

7. The method of claim 2, or of any of claims 3 to 6 when dependent on claim 2, further comprising:

measuring a thermal breakpoint for the hydroprocessed first kerosene fraction;
measuring the one or more of a sulfur content, a nitrogen content, an aromatics content, and an API gravity for the hydroprocessed first kerosene fraction; and
updating the constructed model by incorporating the first kerosene fraction and the hydroprocessed first kerosene fraction into the plurality of kerosene fractions.

8. The method of any of the above claims, wherein the weights for the plurality of compositional groups further comprise at least one weight for a compound.

9. The method of claim 2, or of any of claims 3 to 6 when dependent on claim 2, wherein determining one or more of a sulfur content, a nitrogen content, an aromatics content, and an API gravity for the first kerosene fraction comprises measuring a sulfur content, a nitrogen content, and an API gravity.

10. The method of any of the above claims, wherein measuring or determining a thermal breakpoint further comprises measuring or determining a breakpoint stability.

11. The method of any of the above claims, wherein a thermal breakpoint corresponds to a thermal breakpoint determined according to ASTM D3241.

12. The method of claim 2, or any of claims 3 to 11 when dependent on claim 2, wherein assigning weights for the plurality of compositional groups for the first kerosene fraction comprises assigning weights based on the determined thermal breakpoint and the determined one or more of a sulfur content, a nitrogen content, an aromatics content, and an API gravity, wherein such assigning of weights comprises:

identifying one or more kerosene fractions in the plurality of kerosene fractions that have at least one value for a thermal breakpoint, a sulfur content, a nitrogen content, an aromatics content, or an API gravity that differs from the corresponding value for the first kerosene fraction by less than a threshold value;
determining a weighted average of the one or more identified kerosene fractions, the determined weighted average having weighted average values for a thermal breakpoint and for the at least one of a sulfur content, a nitrogen content an aromatics content, and an API gravity to within a tolerance value; and
calculating weights for the plurality of compositional groups for the first kerosene fraction based on compositional group weights for the determined weighted average of the one or more identified kerosene fractions.

13. The method of claim 2, or of any of claims 3 to 12 when dependent on claim 2, wherein assigning weights for the plurality of compositional groups for the first kerosene fraction comprises analyzing the first kerosene fraction to determine weights for the plurality of compositional groups.

14. The method of any of the above claims, wherein a kerosene fraction comprises a fraction with an initial boiling point of at least about 284°F (140°C) and a final boiling point of less than about 572°F (300°C).

15. The method of any of the above claims, wherein a compositional group in the plurality of compositional groups is defined based on at least one of, and preferably at least two of, a number and type of heteroatoms in a compound, a Z class for a compound, and a molecular weight for a compound.

**Patentansprüche**

1. Verfahren zur Erstellung eines Modells des thermischen Zersetzungspunktes einer Kerosinfraktion, bei dem ein thermischer Zersetzungspunkt einer Vielzahl von Kerosinfraktionen gemessen wird,
ein oder mehrere von Schwefelgehalt, Stickstoffgehalt, Aromatengehalt und API-Dichte für die Vielzahl von Kerosinfraktionen gemessen wird bzw. werden,
die Vielzahl von Kerosinfraktionen unter der Verwendung von Fourier-Transform-Ionen-Zyklotron-Resonanz-Massenspektrometrie analysiert werden, um Massen für eine Vielzahl von Zusammensetzungsgruppen in jeder Kerosinfraktion zu bestimmen, und
ein Modell zur Korrelation eines thermischen Zersetzungspunktes einer Kerosinfraktion mit

a) dem einen oder mehreren von Schwefelgehalt, Stickstoffgehalt, Aromatengehalt und API-Dichte für eine Kerosinfraktion, und
b) Massen für eine Vielzahl von Zusammensetzungsgruppen in einer Kerosinfraktion,

wobei das Modell auf Grundlage
der gemessenen thermischen Zersetzungspunkte für die Vielzahl von Kerosinfraktionen,
der gemessenen Werte für den einen oder die mehreren von Schwefelgehalt, Stickstoffgehalt, Aromatengehalt und API-Dichte für die Vielzahl von Kerosinfraktionen, und
der bestimmten Massen für die Vielzahl von Zusammensetzungsgruppen in der Vielzahl der Kerosinfraktionen erstellt wird.

2. Verfahren zur Herstellung von Kerosinprodukt, bei dem ein Modell des thermischen Zersetzungspunktes einer Kerosinfraktion gemäß Anspruch 1 erstellt wird, bei dem ferner:

ein thermischer Zersetzungspunkt und der eine oder die mehreren von Schwefelgehalt, Stickstoffgehalt, Aromatengehalt und API-Dichte für eine erste Kerosinfraktion bestimmt werden,
aufbauend auf dem bestimmten thermischen Zersetzungspunkt und dem bestimmten einen bzw. den bestimmten mehreren von Schwefelgehalt, Stickstoffgehalt, Aromatengehalt und API-Dichte das Modell zur Zuordnung von Massen für eine Vielzahl von Zusammensetzungsgruppen für die erste Kerosinfraktion verwendet wird,
aufbauend auf den zugeordneten Massen für die Vielzahl von Zusammensetzungsgruppen, dem bestimmten einen bzw. den bestimmten mehreren von Schwefelgehalt, Stickstoffgehalt, Aromatengehalt und API-Dichte und dem erstellten Modell Wasserstoffbehandlungsbedingungen für die erste Kerosinfraktion ausgewählt werden, wobei das Modell Daten, die sich auf Proben von Kerosinfraktionen, die wasserstoffbehandelt worden sind, beziehen und auch eine Beziehung, die beschreibt, wie Zusammensetzungsgruppen auf Grundlage von verschiedenen Wasserstoffbehandlungsbedingungen modifiziert werden, umfasst, und
die erste Kerosinfraktion unter Verwendung der ausgewählten Wasserstoffbehandlungsbedingungen wasserstoffbehandelt wird.

3. Verfahren nach Anspruch 1 oder 2, bei dem die Vielzahl von Kerosinfraktionen von einer einzigen Ölquelle abgeleitet ist.

4. Verfahren nach einem der vorhergehenden Ansprüche, bei dem eine oder mehrere der Vielzahl von Kerosinfraktionen von einer biologischen Quelle, einer vorraffinierten Ölquelle, oder einer Ölquelle, die einem Crackverfahren unterzogen worden ist, abgeleitet ist bzw. sind, oder bei dem eine oder mehrere Kerosinfraktionen in der Vielzahl von Kerosinfraktionen gealterte Kerosinfraktionen umfasst bzw. umfassen, oder eine Kombination davon.

5. Verfahren nach einem der vorhergehenden Ansprüche, bei dem eine oder mehrere Kerosinfraktion(en) in der Vielzahl von Kerosinfraktionen wasserstoffbehandelte Kerosinfraktion(en) ist bzw. sind, wobei eine Vielzahl von der einen oder den mehreren wasserstoffbehandelten Kerosinfraktionen vorzugsweise mit mindestens einer anderen Kerosinfraktion in der Vielzahl von Kerosinfraktionen, die von der selben Ölquelle abgeleitet sind, in Beziehung gesetzt wird.

6. Verfahren nach einem der vorhergehenden Ansprüche, bei dem Messen von einem oder mehreren von Schwefelgehalt, Stickstoffgehalt, Aromatengehalt und API-Dichte für die Vielzahl von Kerosinfraktionen Messen von Schwefelgehalt, Stickstoffgehalt und API-Dichte umfasst.

7. Verfahren nach Anspruch 2 oder einem der Ansprüche 3 bis 6, sofern sich diese auf Anspruch 2 rückbeziehen, bei dem ferner:

ein thermischer Zersetzungspunkt für die wasserstoffbehandelte erste Kerosinfraktion gemessen wird,
der eine oder die mehreren von Schwefelgehalt, Stickstoffgehalt, Aromatengehalt und API-Dichte für die wasserstoffbehandelte erste Kerosinfraktion gemessen wird bzw. werden, und
das erstellte Modell durch Einbeziehen der ersten Kerosinfraktion und der wasserstoffbehandelten ersten Kerosinfraktion in die Vielzahl von Kerosinfraktionen aktualisiert wird.

8. Verfahren nach einem der vorhergehenden Ansprüche, bei dem die Massen für die Vielzahl an Zusammensetzungsgruppen ferner mindestens eine Masse für eine Verbindung umfassen.

9. Verfahren nach Anspruch 2 oder einem der Ansprüche 3 bis 6, sofern sich diese auf Anspruch 2 rückbeziehen, bei dem Bestimmen von einem oder mehreren von Schwefelgehalt, Stickstoffgehalt, Aromatengehalt und API-Dichte für die erste Kerosinfraktion Messen von Schwefelgehalt, Stickstoffgehalt, und API-Dichte umfasst.

10. Verfahren nach einem der vorhergehenden Ansprüche, bei dem Messen oder Bestimmen eines thermischen Zersetzungspunktes ferner Messen oder Bestimmen einer Zersetzungspunktstabilität umfasst.

11. Verfahren nach einem der vorhergehenden Ansprüche, bei dem ein thermischer Zersetzungspunkt einem thermischen Zersetzungspunkt, der gemäß ASTM D3241 bestimmt ist, entspricht.

12. Verfahren nach Anspruch 2 oder einem der Ansprüche 3 bis 11, sofern sich diese auf Anspruch 2 rückbeziehen, bei dem Zuordnen von Massen für die Vielzahl von Zusammensetzungsgruppen für die erste Kerosinfraktion Zuordnen von Massen aufbauend auf den bestimmten thermischen Zersetzungspunkten und dem bestimmten einen oder den bestimmten mehreren von Schwefelgehalt, Stickstoffgehalt, Aromatengehalt und API-Dichte umfasst, dass:

   eine oder mehrere Kerosinfraktion(en) in der Vielzahl von Kerosinfraktionen identifiziert wird bzw. werden, die mindestens einen Wert für einen thermischen Zersetzungspunkt, Schwefelgehalt, Stickstoffgehalt, Aromatengehalt oder API-Dichte aufweist bzw. aufweisen, der bzw. die sich von dem bzw. den entsprechenden Wert(en) für die erste Kerosinfraktion um weniger als einen Schwellenwert unterscheidet bzw. unterscheiden,
   ein gewichteter Mittelwert der einen oder mehreren Kerosinfraktion (en) innerhalb eines Toleranzwertes bestimmt wird, wobei der bestimmte gewichtete Mittelwert gewichtete Mittelwerte für einen thermischen Zersetzungspunkt und für den mindestens einen von Schwefelgehalt, Stickstoffgehalt, Aromatengehalt oder API-Dichte aufweist, und
   Massen für die Vielzahl von Zusammensetzungsgruppen der ersten Kerosinfraktion aufbauend auf den Massen der Zusammensetzungsgruppen der für die eine oder mehreren Kerosinfraktion(en) bestimmten gewichteten Mittelwerte berechnet werden.

13. Verfahren nach Anspruch 2 oder einem der Ansprüche 3 bis 12, sofern sich diese auf Anspruch 2 rückbeziehen, bei dem Zuordnen von Massen für die Vielzahl von Zusammensetzungsgruppen für die erste Kerosinfraktion Analysieren der ersten Kerosinfraktion umfasst, so dass Massen für die Vielzahl von Zusammensetzungsgruppen bestimmt werden.

14. Verfahren nach einem der vorhergehenden Ansprüche, bei dem eine Kerosinfraktion eine Fraktion mit einem unteren Siedepunkt von mindestens etwa 284 °F (140 °C) und einem oberen Siedepunkt von weniger als etwa 572 °F (300 °C) umfasst.

15. Verfahren nach einem der vorhergehenden Ansprüche, bei dem eine Zusammensetzungsgruppe in der Vielzahl von Zusammensetzungsgruppen aufbauend auf mindestens einem und vorzugsweise auf zweien von Anzahl und Art der Heteroatome in einer Verbindung, Z-Klasse einer Verbindung und Molekulargewicht einer Verbindung, definiert wird.

**Revendications**

1. Procédé de construction d'un modèle du point de rupture thermique d'une fraction de kérosène, comprenant :

   la mesure d'un point de rupture thermique pour une pluralité de fractions de kérosène ;
   la mesure d'un ou plusieurs paramètres parmi une teneur en soufre, une teneur en azote, une teneur en aromatiques et une densité API pour la pluralité de fractions de kérosène ;
   l'analyse de la pluralité de fractions de kérosène, par spectrométrie de masse à résonance cyclotronique ionique à transformée de Fourier, pour déterminer des poids pour une pluralité de groupes compositionnels à l'intérieur de chaque fraction de kérosène ; et
   la construction d'un modèle pour corréler un point de rupture thermique d'une fraction de kérosène avec a) le ou les paramètres parmi une teneur en soufre, une teneur en azote, une teneur en aromatiques et une densité API pour une fraction de kérosène, et b) des poids pour une pluralité de groupes compositionnels dans une fraction de kérosène, le modèle étant construit sur la base des points de rupture thermique mesurés pour la pluralité de fractions de kérosène, des valeurs mesurées pour le ou les paramètres parmi une teneur en soufre, une teneur en azote, une teneur en aromatiques et une densité API pour la pluralité de fractions de kérosène, et des poids déterminés pour la pluralité de groupes compositionnels pour la pluralité de fractions de kérosène.

**2.** Procédé de préparation d'un produit à base de kérosène, comprenant la construction d'un modèle du point de rupture thermique d'une fraction de kérosène selon la revendication 1, et comprenant en outre :

la détermination, pour une première fraction de kérosène, d'un point de rupture thermique et du ou des paramètres parmi une teneur en soufre, une teneur en azote, une teneur en aromatiques et une densité API ;
l'utilisation du modèle pour affecter des poids pour une pluralité de groupes compositionnels pour la première fraction de kérosène sur la base du point de rupture thermique déterminé et du ou des paramètres déterminés parmi une teneur en soufre, une teneur en azote, une teneur en aromatiques et une densité API ;
la sélection de conditions d'hydrotraitement pour la première fraction de kérosène sur la base des poids affectés pour la pluralité de groupes compositionnels, du ou des paramètres déterminés parmi une teneur en soufre, une teneur en azote, une teneur en aromatiques et une densité API, et du modèle construit ; le modèle comportant des données relatives à des échantillons de fractions de kérosène qui ont été hydrotraités et comportant également une relation décrivant la façon dont des groupes compositionnels sont modifiés sur la base de diverses conditions d'hydrotraitement ; et
l'hydrotraitement de la première fraction de kérosène au moyen des conditions d'hydrotraitement sélectionnées.

**3.** Procédé des revendications 1 ou 2, dans lequel la pluralité de fractions de kérosène proviennent d'une seule source de pétrole.

**4.** Procédé de l'une quelconque des revendications précédentes, dans lequel une ou plusieurs de la pluralité de fractions de kérosène proviennent d'une source biologique, d'une source de pétrole brut pré-raffinée, ou d'une source de pétrole qui a été exposée à un procédé de craquage, ou dans lequel une ou plusieurs fractions de kérosène dans la pluralité de fractions de kérosène comprennent des fractions de kérosène vieillies, ou une combinaison de celles-ci.

**5.** Procédé de l'une quelconque des revendications précédentes, dans lequel une ou plusieurs fractions de kérosène dans la pluralité de fractions de kérosène sont des fractions de kérosène hydrotraitées, une pluralité de la ou des fractions de kérosène hydrotraitées étant de préférence associées à au moins une autre fraction de kérosène dans la pluralité de fractions de kérosène sur la base de leur provenance de la même source de pétrole.

**6.** Procédé de l'une quelconque des revendications précédentes, dans lequel la mesure d'un ou plusieurs paramètres parmi une teneur en soufre, une teneur en azote, une teneur en aromatiques et une densité API pour la pluralité de fractions de kérosène comprend la mesure d'une teneur en soufre, d'une teneur en azote et d'une densité API.

**7.** Procédé de la revendication 2, ou de l'une quelconque des revendications 3 à 6 lorsqu'elles sont dépendantes de la revendication 2, comprenant en outre :

la mesure d'un point de rupture thermique pour la première fraction de kérosène hydrotraitée ;
la mesure du ou des paramètres parmi une teneur en soufre, une teneur en azote, une teneur en aromatiques et une densité API pour la première fraction de kérosène hydrotraitée ; et
l'actualisation du modèle construit par incorporation de la première fraction de kérosène et de la première fraction de kérosène hydrotraitée dans la pluralité de fractions de kérosène.

**8.** Procédé de l'une quelconque des revendications précédentes, dans lequel les poids pour la pluralité de groupes compositionnels comprennent en outre au moins un poids pour un composé.

**9.** Procédé de la revendication 2, ou de l'une quelconque des revendications 3 à 6 lorsqu'elles sont dépendantes de la revendication 2, dans lequel la détermination d'un ou plusieurs paramètres parmi une teneur en soufre, une teneur en azote, une teneur en aromatiques et une densité API pour la première fraction de kérosène comprend la mesure d'une teneur en soufre, d'une teneur en azote et d'une densité API.

**10.** Procédé de l'une quelconque des revendications précédentes, dans lequel la mesure ou la détermination d'un point de rupture thermique comprend en outre la mesure ou la détermination d'une stabilité au point de rupture.

11. Procédé de l'une quelconque des revendications précédentes, dans lequel un point de rupture thermique correspond à un point de rupture thermique déterminé selon la norme ASTM D3241.

12. Procédé de la revendication 2, ou de l'une quelconque des revendications 3 à 11 lorsqu'elles sont dépendantes de la revendication 2, dans lequel l'affectation de poids pour la pluralité de groupes compositionnels pour la première fraction de kérosène comprend l'affectation de poids sur la base du point de rupture thermique déterminé et du ou des paramètres déterminés parmi une teneur en soufre, une teneur en azote, une teneur en aromatiques et une densité API, une telle affectation de poids comprenant :

   l'identification d'une ou plusieurs fractions de kérosène dans la pluralité de fractions de kérosène qui ont au moins une valeur pour un point de rupture thermique, une teneur en soufre, une teneur en azote, une teneur en aromatiques ou une densité API qui diffère de la valeur correspondante pour la première fraction de kérosène de moins d'une valeur seuil ;
   la détermination d'une moyenne pondérée de la ou des fractions de kérosène identifiées, la moyenne pondérée déterminée ayant des valeurs moyennes pondérées pour un point de rupture thermique et pour l'au moins un paramètre parmi une teneur en soufre, une teneur en azote, une teneur en aromatiques et une densité API à l'intérieur d'une valeur de tolérance ; et
   le calcul de poids pour la pluralité de groupes compositionnels pour la première fraction de kérosène sur la base de poids de groupes compositionnels pour la moyenne pondérée déterminée de la ou des fractions de kérosène identifiées.

13. Procédé de la revendication 2, ou de l'une quelconque des revendications 3 à 12 lorsqu'elles sont dépendantes de la revendication 2, dans lequel l'affectation de poids pour la pluralité de groupes compositionnels pour la première fraction de kérosène comprend l'analyse de la première fraction de kérosène pour déterminer des poids pour la pluralité de groupes compositionnels.

14. Procédé de l'une quelconque des revendications précédentes, dans lequel une fraction de kérosène comprend une fraction avec un point d'ébullition initial au moins d'environ 284 °F (140 °C) et un point d'ébullition final de moins d'environ 572 °F (300 °C).

15. Procédé de l'une quelconque des revendications précédentes, dans lequel un groupe compositionnel dans la pluralité de groupes compositionnels est défini sur la base d'au moins un, et de préférence au moins deux paramètres parmi un nombre et un type d'hétéroatomes dans un composé, une classe Z pour un composé, et un poids moléculaire pour un composé.

**FIG. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7297963 B, MOSES

**Non-patent literature cited in the description**

- **N.J. KUPROWICZ et al.** *Energy & Fuel,* 01 March 2007, vol. 21, 530-544

- **X. CHEN et al.** *Energy & Fuel,* 15 March 2012, vol. 26, 1707-1714